# EUROPEAN PATENT APPLICATION

(11) **EP 4 389 016 A1**
(43) Date of publication of application: **26.06.2024**
(21) Application number: 22215474.2
(22) Date of filing: 21.12.2022
(51) Int. Cl.: A61B 8/08

(54) **IMAGE ACQUISITION METHOD**

(71) Applicant: Koninklijke Philips N.V., 5656 AG Eindhoven (NL)
(72) Inventor: WEBER, Frank Michael, Eindhoven (NL); WAECHTER-STEHLE, Irina, Eindhoven (NL); GESSERT, Nils Thorben, 5656AG Eindhoven (NL); PETERS, Jochen, Eindhoven (NL); LOSSAU, Tanja, Eindhoven (NL); WEHLE, Simon, Eindhoven (NL); GOOßEN, Andre, Eindhoven (NL); WILD, Sebastian, Eindhoven (NL); GROTH, Alexandra, Eindhoven (NL)
(74) Representative: Philips Intellectual Property & Standards

(57) **Abstract**

A method for acquiring a set of target planar views through an anatomy of interest through processing of 3D ultrasound image data. The method processes received 3D image frames in real time to extract target planes and to process each extracted plane with a quality check algorithm. A record is kept of all target planes which have been acquired with sufficient quality, for example by removing them from a list of planes still to be captured. Optionally, each new frame need only be processed to extract planes which are yet to be acquired with sufficient quality. A user interface display is dynamically controlled so that a user is always presented with a real-time display of a planar view of at least one of the planes still to be captured with sufficient quality. The user is thus continually provided the optimal guidance imagery for controlling the acquisition apparatus to acquire new 3D frames which include image data from which the still required 2D planes can be successfully extracted with high quality.

## Description

### FIELD OF THE INVENTION

The present invention relates to the field of medical image acquisition, in particular to methods for user-guided image acquisition.

### BACKGROUND OF THE INVENTION

Within the context of medical imaging, it is often necessary to acquire a standardized set of image views of a certain anatomy. For example, the standard echocardiography protocol includes acquisition of the following views: Parasternal Long Axis, Parasternal Short Axis, Apical 4 Chamber, Subxiphoid (Subcostal), and Inferior Vena Cava view. Fetal ultrasound imaging also involves acquisition of a sequence of views in order to capture standard fetal measurements, e.g. biparietal diameter (BPD), head circumference (HC), abdominal circumference (AC), and femur length (FL).

One approach to doing this is to acquire one or more 3D images and to extract the necessary planes from the 3D image. This has the advantage that a user does not need to precisely position the ultrasound imaging probe for each required planar view. Instead a more limited set of one or more 3D images can be acquired and the necessary view planes extracted as 2D images from the 3D image data.

Methods exist for automatically extracting from a 3D ultrasound image a set of one or more 2D planes.

Within the context of a such as system, it is important that all planes are recorded with a sufficient quality. It is also preferable that image quality be assessed in real-time during the scan so that additional scan data can be acquired if image quality is poor. This avoids suboptimal image quality only becoming apparent after a patient has left.

In some cases, a single 3D image may enable all 2D planes to be extracted with sufficient quality. However, more often, some planes extracted from a given 3D image have adequate quality while others do not. For example, some regions of the acquired 3D image may have poor contrast compared to others, meaning that only a subset of planes can be extracted with high quality. Therefore, in current practice, a user must manually review the quality of all planes sliced from a current 3D volume during the acquisition while they are displayed in real-time. However, given that a multitude of planes are targeted, the user is required to monitor multiple views together during scanning with the aim of optimizing image quality for all at once. This is very challenging since, in practice, a user can only ever focus on one view at a time.

It would be of benefit to provide an ultrasound acquisition method capable of mitigating one or more of the above problems.

### SUMMARY OF THE INVENTION

The invention is defined by the claims.

According to examples in accordance with an aspect of the invention, there is provided a computer-implemented method for use during an ultrasound scan performed by a user with an ultrasound acquisition apparatus.

The method comprises: receiving from the ultrasound acquisition apparatus real-time 3D ultrasound data of an anatomical object, comprising a series of one or more 3D frames, each 3D frame spanning a 3D volume; and determining a plurality of target planes through the 3D volume to be extracted and recording said target planes in a target plane list. The method further comprises performing a plane capturing operation comprising, for each of at least a subset of the 3D frames of the 3D ultrasound data: extracting a selected set of the determined plurality of target planes from the 3D ultrasound data; and controlling a user interface to provide a real-time ultrasound image display output displaying a selected subset (e.g. one) of the extracted set of planes of the current frame; and storing the extracted planes in a plane dataset. The method further comprises, performing, in parallel with the plane capturing operation, a quality assessment operation comprising: applying a pre-determined quality assessment algorithm to planes in the plane dataset to determine, for each target plane in the target plane list, whether any individual extracted plane in the dataset corresponding to the target plane meets a pre-defined quality standard, or whether a time-series of extracted planes in the dataset corresponding to the same target plane meets a pre-defined quality standard, and updating the target plane list to remove from the list any of the target planes which has met the pre-determined quality standard. The method further comprises repeating the steps of the plane capturing operation for each next 3D frame until the target plane list is empty. For each frame, the selected set of target planes to be extracted comprises at least each of the planes still included on the list. For each frame, the selected subset of the extracted planes to be displayed on the user interface includes at least one of the planes still included on the list.

Embodiments of the present invention provide an image acquisition control sequence in which each relevant frame of a 3D ultrasound data stream is processed in real time to extract a set of target anatomical planes, and wherein each of these planes is subjected to a quality assessment to check it meets a quality standard. It is further proposed to retain a list in local memory which records the set of target planes which are yet to meet the defined quality standard. The list can start with all of the target planes to-be-captured included on it, and be updated each time a plane is newly captured which meets the quality criteria. Once the list is empty, all target planes have been captured with sufficient quality.

The quality assessment may be configured to be applied to single planes or configured to be applied to time-series of planes. In some embodiments, a particular target plane may be judged to have been successfully acquired if at least one singular extracted plane corresponding to the target plane has been acquired with a quality which meets a quality standard. In some embodiments, a particular target plane may be judged to have been successfully acquired if a certain time-series of extracted planes corresponding to the target plane (over a series of frames) meets a pre-defined quality standard. For example, the time series may span over a complete heart cycle in the case of cardiac imaging.

With each new 3D frame, in some embodiments, only those target planes which are still included on the target plane list need be extracted from the newly acquired volume. This conserves processing resource and saves time. One advantage provided by the proposed method is to automatically keep track of the target planes which are yet to be captured with sufficient quality and automatically execute extraction, quality check and storage steps to ensure that the full set of target planes is acquired with high quality during the scan. A further advantage of the proposed method is to provide, throughout the scan, a real-time display window which displays a subset (preferably just one) of the extracted planes for a most recent 3D frame, and wherein the display window is continually updated so that it is always showing a plane which is yet to be acquired with a sufficient quality (quality which meets the required quality standard). In this way, the user performing the scan is always being presented a live image of a target view which is still in need of acquisition. Thus the user can focus on adjusting probe placement to optimize quality of this target plane.

Thus, one could understand the proposed method as two processes operating in parallel: one comprising an automatic plane extraction method that operates to automatically extract target planes from a series of 3D frames in such a way that a complete set of target planes is acquired with sufficient quality; and a second comprising presenting a user a display panel on a user interface display which is continually updated to display a live ultrasound image from at least one view for which a target plane has yet to be successfully acquired.

It is noted that the plane capturing operation need not be performed for every frame which is received from the ultrasound acquisition apparatus, but could be performed for every x frames, where x is greater than 1. This could be implemented to save processing resource for example.

The above-reference subset of extracted planes which are displayed on the user interface may preferably be just a single plane. This may be referred to in this document as a `supervised plane' because it is intended to provide a user a real-time imaging view which they can use to adjust the positioning of the ultrasound imaging apparatus or adjust acquisition parameters for example.

The method involves a step of determining the plurality of target planes to be acquired. This could be achieved in some embodiments by simply accessing a database or data list recording the planes to be acquired. In some embodiments, it may comprise identifying the planes through anatomical segmentation.

For example, in some embodiments, the step of determining the plurality of target planes comprises: applying an anatomical segmentation algorithm to at least one of the 3D frames to identify one or more pre-defined anatomical locations within the 3D frame; retrieving a pre-determined list of target views, each target view being associated with an anatomical location; and determining the target planes based on the target views and the segmentation of the 3D frame. Thus, the method extracts planes so as to acquire the target views. The segmentation may be performed for every 3D frame that is processed or could be performed only once and the same target planes used for each subsequent frame.

In some embodiments, the method comprises displaying a visual indication on the user interface of the target planes which remain on the target plane list. This helps guide the user in performing the scan since they can position the ultrasound probe in such a way that optimizes capturing all of the target planes yet to be captured. The visual indication might for example comprise a text list. The visual indication might comprise a graphical representation of the target planes. In some embodiments, the display may include a display area in which the full set of extracted planes are displayed as images in tiles, and wherein each tile is annotated to indicate whether the relevant plane meets the quality standard or does not meet the quality standard.

In some embodiments, the user interface includes a first display region and a second display region, and wherein the method comprises: controlling the user interface to display in the first display region said selected subset (e.g. one) of the plurality of extracted planes with a first image size, and controlling the user interface to display in the second display region a plurality, e.g. all, of the extracted planes, each with a smaller image size than the first image size. For example, the second display region may display the extracted planes as an array of smaller tiles, while the first display region shows a larger image for guiding the user in actively positioning the ultrasound probe.

In some embodiments, the method comprises controlling the user interface to display in the second display region all of the extracted planes of the current frame, each being annotated with a visual indication of whether it remains on the target plane list. For example, the visual indication might be a boundary around the displayed target plane, and wherein a graphical or visual property of the boundary is varied in dependence upon whether the plane is still on the list. For example, the graphical or visual property might be a color of the boundary.

In some embodiments, the quality classification may comprise a graded quality score. In some embodiments, the quality classification may comprise a binary pass/fail classification.

In some embodiments, the method comprises controlling the user interface to provide a user control permitting the user to override the quality classification output from the quality assessment for each extracted plane, for example to thereby manually remove any extracted plane from the list of target planes to be extracted, or to manually re-add any extracted plane to the list of target planes to be extracted.

In other words, it is proposed to control the user interface to provide a user control option permitting a user to manually set the quality classification for a particular plane or series of planes to a pass (or a fail).

In some embodiments, the quality assessment algorithm may be configured to identify extracted planes which pass or fail said quality standard by a margin which is less than a pre-defined threshold margin, and classify said extracted planes or series of planes with a borderline classification. The method may comprise controlling the user interface to display a visual indication of the target planes with a borderline classification, and to prompt a user for user input. The quality classification may be fixed as pass or fail in dependence upon the user input.

In other words, in accordance with this set of embodiments, it is proposed that for images with border-line quality scores (e.g., close to an acceptance threshold), the user may be asked whether they think the quality is sufficient.

The invention can also be embodied in software form. Thus, another aspect of the invention is a computer program product comprising computer program code configured, when run on a processor, to cause the processor to perform a method in accordance with any embodiment or example described in this document or in accordance with any claim of this application.

The invention can also be embodied in hardware form. Thus, another aspect of the invention is a processing device for use with an ultrasound acquisition apparatus during an ultrasound scan performed by a user, the processing device comprising: an input/output; and one or more processors. The one or more processors are configured to: receive from the ultrasound acquisition apparatus real-time 3D ultrasound data of an anatomical object, comprising a series of 3D frames, each 3D frame spanning a 3D volume; and determine a plurality of target planes through the 3D volume to be extracted and recording said target planes in a target plane list.

The one or more processors are further configured to perform a plane capturing operation. The plane capturing operation comprises, for each of at least a subset of the received frame of the 3D ultrasound data: extracting a selected set of the determined plurality of target planes from the 3D ultrasound data; controlling a user interface to provide a real-time ultrasound image display output displaying a selected subset (e.g. one) of the extracted set of planes of the current frame; and storing the extracted planes in a plane dataset.

The one or more processors are further adapted to perform, in parallel with the plane capturing operation, a quality assessment operation comprising: applying a pre-determined quality assessment algorithm to planes in the plane dataset to determine, for each target plane in the target plane list, whether any individual extracted plane in the dataset corresponding to the target plane meets a pre-defined quality standard, or whether a time-series of extracted planes in the dataset corresponding to the same target plane meets a pre-defined quality standard, and updating the target plane list to remove from the list any of the target planes which has met the pre-determined quality standard.

The method further comprises repeating the steps of the plane capturing operation for each next 3D frame of the series of frames until the target plane list is empty.

The quality assessment operation may be repeated at intervals in parallel with the plane capturing operation. It may be run each time the plane capturing operation is run in some examples.

For each frame, the selected set of the determined plurality of target planes to be extracted comprises at least each of the planes still included on the list. For each frame, the selected subset (e.g. one) of the extracted plurality of planes to be displayed on the user interface includes at least one of the planes still included on the list.

Another aspect of the invention is a system, comprising: the processing device according to any embodiment or example described in this document or according to any claim of this application; and an ultrasound acquisition system comprising an ultrasound probe for manipulation by a user. In some embodiments, the system may further comprise a user interface comprising a display unit.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines in block diagram form steps of an example method in accordance with one or more embodiments of the invention;
Fig. 2 is a block diagram showing components of an example processing device and system in accordance with one or more embodiments of the invention;
Fig. 3 illustrates an example display screen of a user interface in accordance with one or more embodiments of the invention;
Fig. 4 shows changes in the generated display screen of an example user interface after each of a series of new 3D image frames is acquired; and
Fig. 5 shows an example ultrasound acquisition apparatus.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method for acquiring a set of target planar views through an anatomy of interest through processing of 3D ultrasound image data. The method processes received 3D image frames in real time to extract target planes and processes the extracted planes, either singularly, or as time-series, with a quality check algorithm. A record is kept of all target planes which have been acquired with sufficient quality, for example by removing them from a list of planes still to be captured. Optionally, each new frame need only be processed to extract target planes which are yet to be acquired with sufficient quality. A user interface display is dynamically controlled so that a user is always presented with a real-time display of a planar view of at least one of the planes still to be captured with sufficient quality. The user is thus continually provided the optimal guidance imagery for controlling the acquisition apparatus to acquire new 3D frames which include image data from which the still required 2D planes can be successfully extracted with high quality.

In other words, it is proposed to provide an acquisition system and method that ensures that for all desired 2D planes, at least one 3D image is recorded from which the 2D plane can be extracted (sliced) with sufficient quality, or at least one time-series of 3D frames of a pre-defined time span (e.g. one complete heart cycle, or one complete phase of the heart cycle) is recorded from which a time-series of frames of the relevant 2D plane (e.g. a cine clip) can be extracted (sliced) with sufficient quality.

According to one particular set of example embodiments, this might be achieved by applying a supervision protocol as follows.

A user may acquire a first 3D ultrasound volume while supervising a reduced set of sliced 2D planes (e.g., only a single plane). Here, `supervising' means that a real-time display image of the reduced set of 2D planes is presented on a user interface display, and the user can utilize this to guide image acquisition. A plane-extraction algorithm may extract (slice) all desired 2D planes from the 3D volume. An automatic quality detection algorithm may analyze the extracted planes to determine which of the sliced 2D planes have a sufficient quality and which do not. Since the user is presented with only a reduced set of the target planes, the number of planes which do not meet the quality criteria may exceed the number of planes which the user is supervising. If all sliced planes have sufficient quality, the acquisition may be complete, and the method may end. If not, then a second 3D ultrasound volume is acquired, while the user interface is updated to now display a subset of the previously extracted 2D planes that did not have sufficient quality when assessed by the quality detection algorithm. In other words, the supervision view is dynamically adjusted to one or more of the target 2D planes which has not yet met the quality criterion. The procedure is repeated (i.e. further 3D volumes are acquired) until all of the target planes can be extracted with sufficient quality from at least one of the acquired 3D frames, and preferably from a time-series of the 3D frames spanning a pre-defined time span, e.g. a complete motion cycle of the imaged anatomical object, e.g. a complete heart cycle.

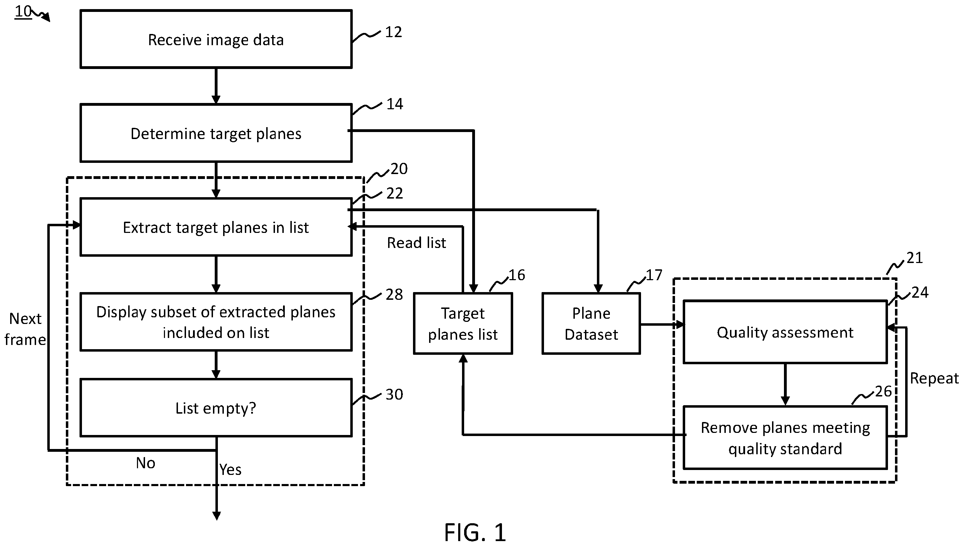

Steps of an example method in accordance with one or more embodiments of the invention are outlined more explicitly in block diagram form in Fig. 1. The steps will be described first in summary form, with more expanded explanation of key features presented subsequently.

The method 10 as presented in Fig. 1 is intended as a computer-implemented method for use during an ultrasound scan performed by a user with an ultrasound acquisition apparatus. The method comprises receiving 12 from the ultrasound acquisition apparatus 3D ultrasound data of an anatomical object. This may be real-time 3D ultrasound data, i.e. live ultrasound data, i.e. a stream of ultrasound data. The 3D ultrasound data may comprise a series of one or more 3D image frames, each 3D frame spanning a 3D volume.

The method further comprises determining 14 a plurality of target planes through the 3D volume to be extracted and recording said target planes in a target plane list 16. The target plane list may be recorded in a local short-term memory. The plurality of target planes may be determined by lookup from a list or database, or may be determined through a computation process, as will be explained later.

The method 10 further comprises performing a plane capturing operation 20 to each of at least a subset of the 3D ultrasound planes. The plane capturing operation may be performed for each frame of the ultrasound data, or a subset of the frames, e.g. every x frames, where x is greater than one. It may be performed for selected frames, e.g. it may simply be repeatedly retriggered each time it finishes so long as the target plane list 16 remains non-empty, and wherein it is applied each time to the most recent 3D ultrasound frame that has been received. It may be performed for user-selected frames, e.g. responsive to a user input, such as from a trigger control, the operation is performed to a most recently received 3D frame.

The plane capturing operation 20 comprises, for each 3D frame to which it is applied:
extracting 22 a selected set of the determined plurality of target planes from the 3D ultrasound data, wherein, for each frame, the selected set of target planes to be extracted comprises at least each of the planes still included on the list 16;
controlling 28 a user interface to provide a real-time ultrasound image display output displaying a selected subset of the extracted set of planes of the current frame, wherein, for each frame, the selected subset of the extracted planes to be displayed on the user interface includes at least one of the planes still included on the list 16;
storing the extracted planes in a plane dataset 17; and
checking 30 whether the target plane list 16 is empty and repeating the steps of the plane capturing operation 20 for a next 3D frame of the series of frames if the target plane list 16 is not empty.

In parallel with the plane capturing operation 20, the method also comprises performing a quality assessment operation 21 comprising:
applying 24 a pre-determined quality assessment algorithm to planes in the plane dataset 17 to determine, for each target plane in the target plane list, whether any individual extracted plane in the dataset corresponding to the target plane meets a pre-defined quality standard, or whether a time-series of extracted planes in the dataset corresponding to the same target plane meets a pre-defined quality standard, and
updating 26 the target plane list to remove from the list any of the target planes which has met the pre-determined quality standard.

The steps of the plane capturing operation 20 may be repeated for each next 3D frame of the series of frames until the target plane list is empty,

The quality assessment operation 21 may be executed repeatedly, for example on a loop, for example until the target plane list 16 is empty.

The quality assessment operation 21 may be run at intervals, in parallel with the plane extraction procedure. For convenience, it might be triggered each time the plane extraction operation is triggered, for instance running just before or just after each iteration of the plane extraction procedure. Alternatively, it could be run at different intervals, entirely independent of the plane extraction operation. Its results feed into the plane extraction operation by way of the update of the target plane list which is the output action of the quality assessment operation.

As will be apparent from the above outline, the purpose of the quality assessment operation 21 is to review the planes that have been extracted from the series of 3D frames to check that, for each of the target planes, a defined quality criterion in relation to the target plane has been met. The plane extraction operation 20 may operate on a loop, for a series of received 3D frames, each time extracting the set of target planes from the 3D data and recording these in the plane dataset 17, along with a time-stamp corresponding to the time of acquisition. This plane capturing operation may proceed until the target plane list is empty. At the same time, in parallel, the quality assessment operation 21 may routinely or continuously review the extracted planes dataset 17 that has been so far compiled and check whether, for each target plane still listed in the target plane list 16, a pre-defined quality standard/criterion is met by the set of extracted planes in the plane dataset. In some cases this may comprise checking, for each target plane, whether at least one of the extracted planes meets a pre-defined quality standard. In other embodiments, it may comprise checking whether, for each target plane, a certain time-sequence of frames of the target plane has been acquired which meets a certain quality standard. The planes or series of planes which do meet the quality standard may be tagged or annotated for easy identification later. In some embodiments, a final plane dataset may be compiled as a further step, wherein a dataset comprising a single instance of each of the target planes (or a time-series thereof) is compiled from those extracted planes in the plane dataset 17 which are tagged as having met the quality standard.

In some embodiments, the decision as to whether a target plane has been satisfactorily acquired could be assessed on the level of a complete heart cycle. Thus, the quality assessment 24 for each target plane comprises determining whether a sliced time series of the target frame over a complete heart cycle matches a quality criterion.

Thus, the aforementioned quality assessment algorithm may be configured to receive as input a single frame, or in other embodiments may receive as input a time-series of planes.

The user interface may comprise a display. In some embodiments, a first subregion of the display is used to display the aforementioned subset of the extracted planes (which may be referred to in this document as `supervised planes') in a high resolution (e.g. as large images). In some embodiments, a second subregion of the display is used to display a larger set (e.g. all) of the extracted planes in a lower resolution (e.g. as smaller images). In some embodiments, the smaller images of the planes may be further annotated with a visual status indicator indicating whether the plane has already been extracted with sufficient quality from one of the acquired 3D volumes.

As noted above, the method can also be embodied in hardware form, for example in the form of a processing device which is configured to carry out a method in accordance with any example or embodiment described in this document, or in accordance with any claim of this application.

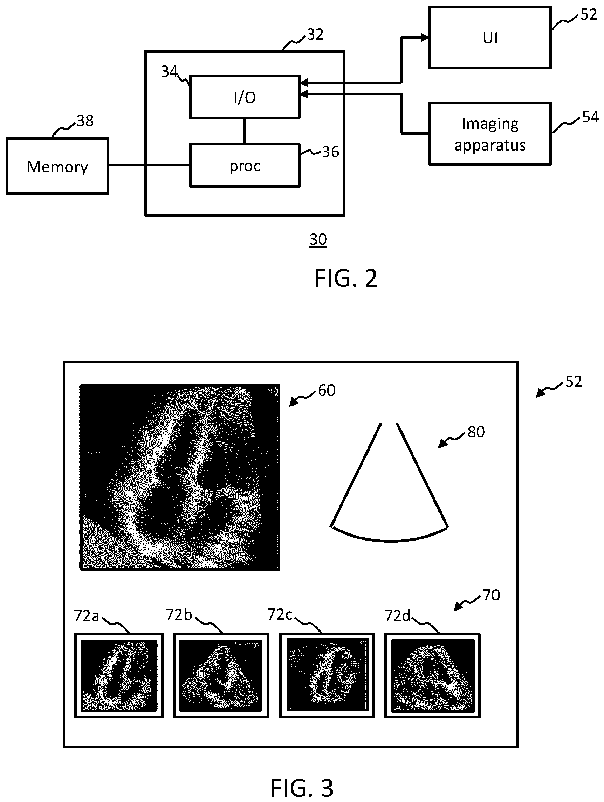

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52. The user interface may comprise a display.

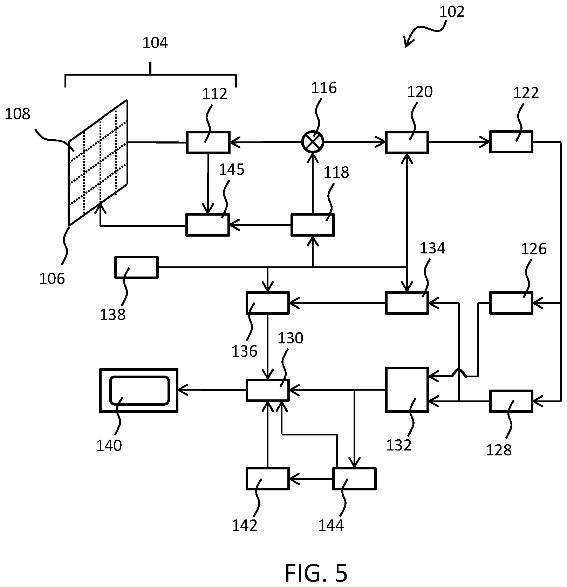

The system 30 further comprises an ultrasound acquisition or imaging apparatus 54 for acquiring 3D ultrasound imaging data 44. Details of an example ultrasound acquisition apparatus 54 are described later with reference to Fig. 5.

In some embodiments, the user interface 52 may be integrated with the ultrasound acquisition apparatus 54. For example, the display of the ultrasound acquisition apparatus may be used as the display of the user interface 52. In further embodiments, the user interface may comprise a display unit which is separate from the ultrasound acquisition apparatus 54.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

With regards to the ultrasound acquisition apparatus 54, this may comprise an ultrasound transducer unit, such as an ultrasound probe, comprising a plurality of ultrasound transducers, e.g. an array transducer. The probe may be a handheld probe in some embodiments. The probe may be a transesophageal (TEE) probe in some examples. The probe may be transthoracic echocardiogram (TTE) probe in some examples.

As mentioned above, it is not essential that the system 30 itself comprise an ultrasound acquisition apparatus. Instead the processing device 32 may be adapted to communicatively couple during use with an ultrasound acquisition apparatus, for example via a network link, such as a DNL link, or for example via an internet connection. Thus, in some embodiments, the ultrasound acquisition apparatus could be remote from the actual processing device which implements the method.

As mentioned above, the method 10 includes a step of initially determining a plurality of target planes through the 3D volume to be extracted and recording said target planes in the target plane list 16. In other words, the method comprises compiling an initial version of the target plane list 16. In some embodiments, determining the plurality of target planes comprises accessing a pre-stored list or database which records the intended target planes. In some embodiments, determining the plurality of target planes may comprise a step of processing one or more of the acquired 3D images with one or more anatomical segmentation algorithms to identify the target planes.

For example, in accordance with at least one set of example embodiments, the step of determining the plurality of target planes may comprise: retrieving a pre-determined list of target views, each target view being associated with an anatomical location; applying an anatomical segmentation algorithm to at least one of the 3D frames to identify one or more pre-defined anatomical locations within the 3D frame; and determining the plurality of target planes based on the target views and the segmentation of the 3D frame.

The application of the segmentation establishes anatomical context from the 3D volume. For example, the segmentation may employ use of model-based segmentation. At least one suitable example method of model-based segmentation is described in the paper: Ecabert, O et al. Automatic Model-Based Segmentation of the Heart in CT Images Medical Imaging, IEEE Transactions on, 2008, 27, pp. 1189-1201.

By way of further example, the segmentation may employ use of a machine-learning based segmentation algorithm, for example a convolutional neural network trained for performing segmentation. At least one suitable machine learning model for performing segmentation is described in: LI, Yuanwei, et al. Standard plane detection in 3d fetal ultrasound using an iterative transformation network. In: International Conference on Medical Image Computing and Computer-Assisted Intervention. Springer, Cham, 2018. S. 392-400.

Based on the anatomical context provided by the segmentation, the method comprises determining the location of a defined set of 2D views within the 3D volume. For example, locations of standard view planes might be encoded into a segmentation model by assigning labels to one or more segmentable anatomical elements that are situated within the view plane. The segmentation results provide the set of segmentable elements labelled as to their location in the 3D frame volume. Linear regression may then be performed through the segmented elements to determine the closest fit planes through the 3D frame volume which match the standard view planes.

Once the target planes have been identified, extraction of the planes from a 3D frame can be performed by slicing through the 3D frame. Extracted planes may be stored in the plane dataset 17.

With regards the aforementioned pre-determined list of target views, these may for example correspond to a set of standard view planes in accordance with a standardized imaging protocol. For example, in the field of cardiac imaging, a standard echocardiogram protocol includes acquisition of the following views: Parasternal Long Axis, Parasternal Short Axis, Apical 4 Chamber, Subxiphoid (Subcostal), and Inferior Vena Cava view. A further, simpler, example is a protocol which comprises the apical 4 chamber view, apical 2 chamber view, an aortic valve view, and a mitral valve view, wherein all of these are acquired from a single fixed probe position, e.g. apical location.

By way of further example, fetal ultrasound imaging involves acquisition of a sequence of views in order to capture standard fetal measurements, e.g. biparietal diameter (BPD), head circumference (HC), abdominal circumference (AC), and femur length (FL).

In some embodiments, the user interface may be controlled to provide the user with a control option permitting a user to select between a plurality of possible sets of target views, e.g. each associated with a standard imaging protocol. These might be referred to as presets. Thus, in this example, the user is able to select from multiple presets, each resulting in a defined set of 2D view planes to be sliced. In some embodiments, the user interface may be controlled to permit a user to freely define a set of target views, e.g. by selection from a database or dataset of pre-defined possible target views.

As discussed above, the proposed method involves applying a pre-determined quality assessment algorithm to planes in the plane dataset 17 to determine, for each target plane in the target plane list, whether a pre-defined quality criterion for the target plane is met by the planes in the plane dataset. In some embodiments, the quality assessment algorithm is configured to determine whether any individual extracted plane in the dataset corresponding to each given target plane meets a pre-defined quality standard. In some embodiments, the quality assessment algorithm is configured to determine, for each target plane in the target plane list, whether a time-series of extracted planes in the dataset corresponding to the same target plane (i.e. a series of frames of the plane) meet a pre-defined quality standard.

In some embodiments, the quality assessment algorithm may be configured to receive a sliced plane, or a time series of sliced planes, together with metadata indicating which target anatomical view plane it represents (e.g. two-chamber view, four-chamber view). By means of imaging processing, the quality assessment algorithm provides a determination as to whether the sliced plane, or time series of planes, has sufficient image quality.

By way of illustration, for the case where the quality assessment is applied to a single plane, the quality assessment algorithm might, for example, be based on analysis of a degree of visibility of one or more anatomical structures (e.g. walls, valves, chambers), or degree of correspondence between the view represented by the plane and the view of a target plane, or a quality level of contrast or noise levels of the image.

For the case where the quality assessment algorithm is applied to a time-series of planes of a same target plane view, the quality assessment algorithm might be based on similar factors assessed for each plane in the time series to check that all planes in the series are of acceptable quality. For example, each plane in the series may be assessed to determine a degree of visibility of one or more anatomical structures (e.g. walls, valves, chambers), or a degree of correspondence between the view represented by the plane and the view of a target plane, or a quality level of contrast or noise levels of the image. In other examples, quality criteria specific to the time series might be assessed, for example absence of motion artefacts, or spatial registration of the series of planes.

The output of the image quality assessment algorithm may in some embodiments include a binary yes/no or pass/fail classification or decision indicating whether the plane or series of planes meets the quality standard. Additionally or alternatively, the output may include a non-binary rating, such as a graded quality score, to which a threshold might be applied to determine whether the pre-defined quality standard is met. If the output score is equal to or above the threshold, the quality is sufficient. If the quality is below the threshold, the quality is not sufficient.

In some embodiments, the quality assessment algorithm may be a machine learning algorithm such as an artificial neural network.

By way of one example, the quality assessment algorithm may comprise a trained convolutional neural network (CNN). The CNN might be trained using a training dataset comprising sliced images, or time sequences of images, each annotated with a corresponding quality score. The quality score annotations may be based on manually assessed quality determined by human experts.

A CNN can be trained to a provide a binary output classification (e.g. pass/fail) or can be trained to provide a graded classification score.

The CNN could be differently trained to generate a score which relates to different possible features of the input image planes. For example, the CNN might be trained to output a score indicative of how well certain anatomical structures (e.g. walls, valves) are visible in the image, or of how closely the plane matches a target plane, or of a quality of contrast/noise levels of the image.

For assessing a quality of a time-series of planes, one option is to apply the above-discussed CNN to each plane in the series, derive a quality classification for each plane, determine whether each plane meets an individual quality standard, and wherein it is determined that the whole series meets the quality standard if all planes constituting the series meet their individual quality standards.

Another option may be to train a CNN to receive as input a series of planes and to determine a quality classification based on factors which pertain to the series, for example proper registration/alignment between successive planes in the series.

In either case, the CNN may be trained with a supervised training procedure to predict the quality scores based on input images. The score prediction can be realized as a regression or a classification problem. A segmentation-based approach is also possible but may not be preferred due to the greater requirements as to annotation.

After training, the CNN can be applied to sliced images during a scanning session in real time. In some embodiments, if the output of the CNN is a graded score, this score may be compared against a threshold to determine whether the relevant extracted plane or series of planes meets a pre-defined quality standard. Alternatively, if the output of the CNN is a binary pass/fail classification, then the output of the algorithm directly provides the indication as to whether the extracted plane or series of planes meets the quality standard.

In some embodiments, a CNN may be trained to generate a plurality of different quality scores, each relating to a different feature of the input image or series of images (examples mentioned above). The different scores produced by the CNN can either be used directly, or might be fused e.g. by averaging or weighted averaging, into a single quality score.

A part of the method involves the control of the user interface 52 to provide a display output to the user that both advises the user as to which of the target planes have already been acquired at sufficient quality and also provides image acquisition guidance through display of a real-time image view corresponding to at least one of the target planes/views yet to be captured with sufficient quality.

An example display output of a user interface 52 in accordance with at least one set of embodiments is depicted in Fig. 3.

There are different display regions provided on the display output.

A first display region 60 provides a real-time ultrasound image display output displaying a selected subset of the extracted set of planes of the current frame. In this example, the selected subset consists of a single one of the extracted set of planes of the current frame. In particular, a single one of the extracted planes which has not yet met the pre-defined quality standard is displayed. In other words, the first display region 60 displays a current image plane which is still recorded on the target plane list 16. The first display region displays the subset of the extracted planes in high resolution, or in a larger size, compared to images presented in a second display region 70. The function of the first display region is to guide the operator in the continued ultrasound scanning procedure. The operator can look at the first display region and adjust the positioning of the ultrasound probe to try to acquire a new 3D image frame which will capture the target plane depicted in the first display region with sufficient quality. Because the method is operating in real time, new 3D frames are being acquired at relatively high frequency, e.g. multiple times per second. Thus, the image depicted in the first display region is continuously updating, always showing an extracted image plane from a latest 3D frame. In this way, the first display region provides a real-time display output for guiding the image acquisition. In effect, the first display region provides a real-time ultrasound image output, characterized in that the particular 2D planar view shown always corresponds to one of the target planes still left to capture at sufficient quality, i.e. one of the target planes still recorded in the target plane list 16. The image plane(s) depicted in the first image region might be referred to as the "supervised" plane(s), permitting the user to easily supervise image quality of these plane(s) during the acquisition.

The display output in the example of Fig. 3 further comprises a second display region 70 which is controlled to display all of the extracted planes, or at least a subset thereof, in a lower resolution and/or at a smaller size. By way of illustration, Fig. 3 shows an example where there are four target planes to acquire in total 72a, 72b, 72c, 72d. Thus the second display region is controlled to display a plurality, e.g. all, of the extracted planes, each with a smaller image size than the image size of the image shown in the first display region 60.

Optionally, the display output of the user interface 52 may further be controlled to include one or more additional display regions. By way of example, as illustrated in Fig. 3, the display output may include a third display region 80 which depicts a volume rendering of an anatomical object or feature located within a current 3D frame.

To aid in understanding of the overall process flow of the method, an example method flow will now be described.

At a start of the scan, the target plane list 16 (see Fig. 1) is loaded with a record of the full list of target planes which are to be captured during the scan.

A user acquires a first 3D ultrasound frame using the ultrasound acquisition apparatus. For the first frame, an image processing algorithm slices from the frame all of the target planes recorded in the target plane list 16. The user interface 52 is controlled to display in the first display region 60 a reduced subset of the extracted planes, for example a single extracted plane. This provides the user with a real-time 'supervised' plane which the user can focus on to guide the continued acquisition of ultrasound image data, i.e. to a guide acquisition of a next 3D frame.

A quality assessment algorithm is applied to the extracted 2D planes. In this described embodiment, the quality assessment is applied to individual extracted planes (for a single time point). As has been discussed above, in further embodiments, the quality assessment could be applied to temporal series of extracted planes. An example implementation according to the latter option will be further discussed later.

Continuing with the present example, the quality assessment algorithm analyzes which of the sliced 2D planes have a sufficient quality (i.e. meet a pre-defined quality standard) and which do not. If all planes extracted from the first 3D frame have sufficient quality, the acquisition is complete. This may be communicated to the user via the user interface.

If not, then the target plane list 16 is updated to remove from the list all planes which have been acquired with sufficient quality. A record may be kept indicating, for each target plane which has been acquired with sufficient quality, the particular 3D frame from which the acceptable quality plane was extracted.

A second 3D ultrasound volume is acquired. This is processed to extract at least each of the planes which are still recorded on the target plane list (i.e. which have not yet been captured with sufficient quality). The supervised plane shown in the first display region 60 of the user interface is updated to show one of the newly extracted planes which is still on the target plane list. Thus, the user is always supervising an image view which is yet to be acquired with sufficient quality. In other words, the supervision view 60 is dynamically adjusted to one or more of the missing 2D planes.

The procedure is repeated (i.e. further 3D volumes are acquired) until all of the target planes originally loaded into the target plane list 16 can be sliced with sufficient quality from at least one of the acquired 3D volumes.

If a 2D plane can be sliced from more than one of the acquired 3D frames, the result of the quality assessment may be used to select the 3D volume from which the plane can be sliced with the best quality (e.g. the volume for which the quality score of the assessment was largest for the relevant plane).

To further guide the user, the method may further comprise displaying a visual indication on the user interface 52 of the target planes which remain on the target plane list 16.

By way of example, the small images of the extracted planes presented in the second display region 70 may include a status indicator which indicates whether the plane has been extracted with sufficient quality. In other words, the method may comprise controlling the user interface to display in the second display region 70 all of the extracted planes of the current frame, each being annotated with a visual indication of whether it remains on the target plane list. For example, the visual indication might be a boundary around the displayed target plane, and wherein a graphical or visual property of the boundary is varied in dependence upon whether the plane is still on the list. For example, the graphical or visual property might be a color of the boundary. In further examples, the visual indicator might be a colored flag presented adjacent to each depicted image plane, or a traffic light (e.g. green, yellow, red).

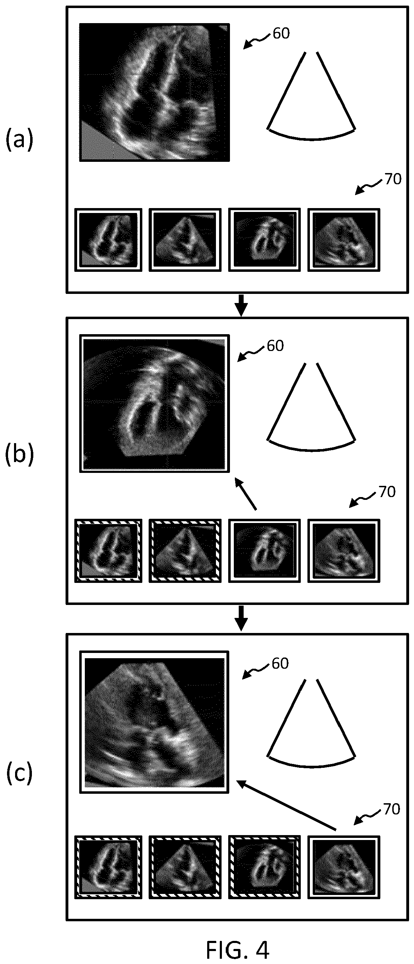

This is indicated schematically in Fig. 4 which shows a display output of a user interface 52 as updated for each of three successive 3D frames. These frames may be directly temporally successive frames acquired by the ultrasound acquisition system or, in some examples, may be frames whose acquisition is manually triggered by a user by means of a user input device, such as a trigger control, e.g. provided on the ultrasound probe. For example, the user may manually trigger a new frame acquisition after adjusting a positioning of the probe. In some examples, the method comprises receiving a real-time series of 3D frames, but wherein the place extraction operation is only applied to a subset of these received planes, and wherein the subset is determined by user input. For example, the user may operate a user control when the user would like a current frame to be passed to the plane extraction operation.

Fig. 4(a) shows a display output of the user interface for a first acquired 3D frame. The first display region shows one of the extracted image planes, and the second display region 70 shows all of the extracted image planes. At this stage, none of the extracted planes has yet been acquired with a quality which meets the pre-defined quality standard. Thus, a next 3D frame is acquired. Upon extraction of all of the target planes from the second 3D frame, two of the extracted planes are now found to meet the pre-defined quality standard, as assessed by the quality assessment algorithm. As shown in Fig. 4(b), the second display region 70 again displays all of the extracted planes, but now displays the two extracted planes which meet the quality standard with a boundary highlight. In the illustration of Fig. 4(b), this is shown by a cross-hatched boundary. The first display region 60 is also updated so that this shows one of the remaining two target planes which has not yet been extracted with sufficient quality. A next 3D frame is then acquired, and at least the two target planes left to be extracted with sufficient quality are extracted. Of these, a further one plane is found to meet the quality standard, and thus, as shown in Fig. 4(c), the second display region 70 is updated to add a boundary highlight to the extracted plane which meets the quality standard. The first display region 60 is updated to display the last remaining plane yet to be extracted with sufficient quality. The method continues until all target planes have been extracted with a quality which passes the quality standard.

In some embodiments, the user interface 52 may be controlled to permit the user to select which of the target planes is displayed in the first display region 60. By way of example, the user might choose the plane whose quality score is currently the lowest. By way of example, the status indicator applied to each of the extracted planes in the second display area 70 may indicate a value of the quality score, e.g. with different colors, or with a number.

In some embodiments, a priority order may be defined in relation to the target planes in the target plane list 16. This might be included as part of the initially loaded target plane list at a start of the scan. In some embodiments, during the scan, the plane displayed in the first display region 60 may be determined according to the priority order, wherein, of the planes still remaining in the target plane list 16 at a given time, the one highest in the priority order is displayed in the first display region. In this way, the user is guided in acquiring more important planes first, i.e. more important planes are supervised first. In this way, in the event that the scan needs to be aborted before completion (for example, due to the condition of the patient), the most important planes have been acquired first.

As mentioned above, as a variation to the above-described example, the quality assessment in respect of a certain target plane/view may be applied at the level of a time-series of extracted planes corresponding to that target plane, spanning a time period. The time period may be defined according to one or more characteristic anatomical events, e.g. a motion cycle of the imaged anatomical object. For example, it is common in the context of cardiac ultrasound imaging to acquire 4D imaging datasets comprising a time-series of 3D image frames which spans one or more complete heart cycles, or one or more instances of a certain phase of the heart-cycle, e.g. systole or diastole. In some embodiments, the method may comprise acquiring a sequence of 3D frames spanning at least one heart cycle, and applying the plane extraction operation 20 to each 3D frame of the sequence to acquire for each frame the set of target planes still included in the target plane list 16. The same set of target planes are extracted from each frame of the sequence. Following this, the quality assessment procedure 21 is then applied to the extracted 2D planes from the sequence of 3D frames spanning the whole time period. In this example, the quality assessment algorithm is configured to receive a time-series of images for each of the target planes. For each target plane, the respective extracted time-series of planes corresponding to this target plane are processed by the quality assessment algorithm to determine if it meets a quality standard. If so, the target plane list 16 can be updated to remove the respective target plane. Thus, this embodiment differs from that described above only in that the quality assessment is performed at the level of a series of planes for an extended time period rather than a single plane for a single time point. By way of example, the quality assessment operation 20 may be triggered in this case after each time-series of 3D frames has been acquired and after the plane extraction operation 20 has been applied to the respective time-series.

In some embodiments, the method may comprise controlling the user interface to provide a user control permitting the user to override the quality classification output from the quality assessment for each extracted plane or series of planes (as appropriate), and to thereby manually add or remove any extracted plane from the list of target planes to be extracted.

Thus, the user is permitted to overrule one or more decisions of the quality assessment algorithm, e.g. to thereby manually set a quality pass/fail classification to a pass (or vice versa), and optionally updating the quality indicator associated with the plane or series or planes in the second display region 70, e.g. to green. The first display region 60 may then be updated with display of a next one of the extracted planes still on the target plane list.

This may be useful in cases for example where the user disagrees with the automatically determined quality classification of one or more of the extracted planes e.g. if the user prefers a somewhat different definition of a 4-chamber view, but nevertheless wants the system to automatically control the quality of the remaining views.

In some embodiments, the method may comprise controlling a user interface to generate a prompt asking a user to confirm whether a quality of one or more of the extracted planes, or series of planes, is considered sufficient. For example, this might be done for planes whose quality score output from the quality assessment algorithm is borderline (e.g., close to an acceptance threshold). The user in this case may be asked whether they think the quality is sufficient.

By way of example, in some embodiments, the quality assessment algorithm is configured to identify extracted planes or series of planes which pass or fail said quality standard by a margin which is less than a pre-defined threshold margin and classify said extracted planes with a borderline classification. The method may comprise controlling the user interface to display a visual indication of the planes with a borderline classification, and to prompt a user for user input. The quality classification may then be fixed as pass or fail in dependence upon the user input.

As noted above, certain embodiments may include an ultrasound acquisition apparatus 54, and/or means for processing ultrasound echo data to derive further data.

By way of further, more detailed explanation, the general operation of an exemplary ultrasound acquisition apparatus will now be described, with reference to Fig. 5.

The system comprises an array transducer probe 104 which has a transducer array 106 for transmitting ultrasound waves and receiving echo information. The transducer array 106 may comprise CMUT transducers; piezoelectric transducers, formed of materials such as PZT or PVDF; or any other suitable transducer technology. In this example, the transducer array 106 is a two-dimensional array of transducers 108 capable of scanning either a 2D plane or a three dimensional volume of a region of interest. In another example, the transducer array may be a 1D array.

The transducer array 106 is coupled to a microbeamformer 112 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is in general entirely optional. Further, the system includes a transmit/receive (T/R) switch 116, which the microbeamformer 112 can be coupled to and which switches the array between transmission and reception modes, and protects the main beamformer 120 from high energy transmit signals in the case where a microbeamformer is not used and the transducer array is operated directly by the main system beamformer. The transmission of ultrasound beams from the transducer array 106 is directed by a transducer controller 118 coupled to the microbeamformer by the T/R switch 116 and a main transmission beamformer (not shown), which can receive input from the user's operation of the user interface or control panel 138. The controller 118 can include transmission circuitry arranged to drive the transducer elements of the array 106 (either directly or via a microbeamformer) during the transmission mode.

The function of the control panel 138 in this example system may be facilitated by an ultrasound controller unit according to an embodiment of the invention.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated, and the process repeated until all the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 118 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 118 can be coupled to control a DC bias control 145 for the transducer array. The DC bias control 145 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 112 and are then passed to a main receive beamformer 120 where the partially beamformed signals from individual patches of transducers are combined into a fully beamformed signal, referred to as radio frequency (RF) data. The beamforming performed at each stage may be carried out as described above, or may include additional functions. For example, the main beamformer 120 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 122. The signal processor 122 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 5 only the receiver beamformers 112, 120 are shown, for simplicity. In the complete system, there will also be a transmission chain with a transmission micro beamformer, and a main transmission beamformer.

The function of the micro beamformer 112 is to provide an initial combination of signals in order to decrease the number of analog signal paths. This is typically performed in the analog domain.

The final beamforming is done in the main beamformer 120 and is typically after digitization.

The transmission and reception channels use the same transducer array 106 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 126 and a Doppler processor 128. The B mode processor 126 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 128 processes temporally distinct signals arising from tissue movement and blood flow for the detection of moving substances, such as the flow of blood cells in the image field. The Doppler processor 128 typically includes a wall filter with parameters set to pass or reject echoes returned from selected types of materials in the body.

The structural and motion signals produced by the B mode and Doppler processors are coupled to a scan converter 132 and a multi-planar reformatter 144. The scan converter 132 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 140. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 142 converts the echo signals of a 3D data set into a projected 3D image as viewed from a given reference point as described in US Pat. 6,530,885 (Entrekin et al.).

The 2D or 3D images are coupled from the scan converter 132, multi-planar reformatter 144, and volume renderer 142 to an image processor 130 for further enhancement, buffering and temporary storage for optional display on an image display 140. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 128 and tissue structure information produced by the B mode processor 126 are coupled to a quantification processor 134. The quantification processor produces measures of different flow conditions such as the volume rate of blood flow in addition to structural measurements such as the sizes of organs and gestational age. The quantification processor may receive input from the user control panel 138, such as the point in the anatomy of an image where a measurement is to be made.

Output data from the quantification processor is coupled to a graphics processor 136 for the reproduction of measurement graphics and values with the image on the display 140, and for audio output from the display device 140. The graphics processor 136 can also generate graphic overlays for display with the ultrasound images. These graphic overlays can contain standard identifying information such as patient name, date and time of the image, imaging parameters, and the like. For these purposes, the graphics processor receives input from the user interface 138, such as patient name. The user interface is also coupled to the transmit controller 118 to control the generation of ultrasound signals from the transducer array 106 and hence the images produced by the transducer array and the ultrasound system. The transmit control function of the controller 118 is only one of the functions performed. The controller 118 also takes account of the mode of operation (given by the user) and the corresponding required transmitter configuration and band-pass configuration in the receiver analog to digital converter. The controller 118 can be a state machine with fixed states.

The user interface is also coupled to the multi-planar reformatter 144 for selection and control of the planes of multiple multi-planar reformatted (MPR) images which may be used to perform quantified measures in the image field of the MPR images.

The above described ultrasound system may be operatively coupled with the processing device 32 previously described. For example, the above-described ultrasound system may be used to implement the ultrasound acquisition apparatus 54 of the system 30 shown in Fig. 2 in some examples.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method (10) for use during an ultrasound scan performed by a user with an ultrasound acquisition apparatus, comprising:
receiving (12) from the ultrasound acquisition apparatus real-time 3D ultrasound data of an anatomical object, comprising a series of one or more 3D frames, each 3D frame spanning a 3D volume;
determining (14) a plurality of target planes through the 3D volume to be extracted and recording said target planes in a target plane list (16);
performing a plane capturing operation (20) comprising, for each of at least a subset of the 3D frames of the 3D ultrasound data:
extracting (22) a selected set of the determined plurality of target planes from the 3D ultrasound data,
controlling (28) a user interface to provide a real-time ultrasound image display output displaying a selected subset of the extracted set of planes of the current frame, and
storing the extracted planes in a plane dataset (17);
performing, in parallel with the plane capturing operation, a quality assessment operation (21) comprising:
applying (24) a pre-determined quality assessment algorithm to planes in the plane dataset to determine, for each target plane in the target plane list, whether any individual extracted plane in the plane dataset corresponding to the target plane meets a pre-defined quality standard, or whether a time-series of extracted planes in the plane dataset corresponding to the same target plane meets a pre-defined quality standard, and
updating (26) the target plane list to remove from the list any of the target planes which has met the pre-defined quality standard;
repeating the steps of the plane capturing operation for each next 3D frame of the series of frames until the target plane list is empty,
wherein, for each frame, the selected set of target planes to be extracted comprises at least each of the planes still included on the target plane list;
wherein, for each frame, the selected subset of the extracted planes to be displayed on the user interface includes at least one of the planes still included on the target plane list.

2. The method of claim 1, wherein determining the plurality of target planes comprises:
applying an anatomical segmentation algorithm to at least one of the 3D frames to identify one or more pre-defined anatomical locations within the 3D frame;
retrieving a pre-determined list of target views, each target view being associated with an anatomical location;
determining the target planes based on the target views and the segmentation of the 3D frame.

3. The method of claim 1 or 2, further comprising displaying a visual indication on the user interface of the target planes which remain on the target plane list.

4. The method of any of claims 1-3, wherein said selected subset of the extracted plurality of planes to be displayed on the user interface consists of only a single one of the extracted plurality of planes.

5. The method of any of claims 1-4,
wherein the user interface includes a first display region and a second display region; and
wherein the method comprises:
controlling the user interface to display in the first display region said selected subset of the plurality of extracted planes with a first image size, and
controlling the user interface to display in the second display region a plurality, e.g. all, of the extracted planes, each with a smaller image size than the first image size.

6. The method of claim 5, wherein the method comprises controlling the user interface to display in the second display region all of the extracted planes of the current frame, each being annotated with a visual indication of whether it remains on the target plane list.

7. The method of any of claims 1-6, wherein the quality classification comprises a graded quality score, or wherein the quality classification is a binary pass/fail classification.

8. The method of any of claims 1-7, wherein the method comprises controlling the user interface to provide a user control permitting the user to override the quality classification output from the quality assessment for each extracted plane or time-series of planes, and to thereby manually add or remove any extracted plane from the target plane list.

9. The method of any of claims 1-8,
wherein the quality assessment algorithm is configured to identify extracted planes or time-series of planes which pass or fail said quality standard by a margin which is less than a pre-defined threshold margin, and classify said extracted planes with a borderline classification;
wherein the method comprises controlling the user interface to display a visual indication of the planes with a borderline classification, and to prompt a user for user input;
wherein the quality classification is fixed as pass or fail in dependence upon the user input.

10. A computer program product comprising computer program code configured, when run on a processor, to cause the processor to perform a method in accordance with any of claims 1-9.

11. A processing device (32) for use with an ultrasound acquisition apparatus (54) during an ultrasound scan performed by a user, the processing device comprising:
an input/output (34); and
one or more processors (36), configured to:
receive from the ultrasound acquisition apparatus real-time 3D ultrasound data of an anatomical object, comprising a series of 3D frames, each 3D frame spanning a 3D volume;
determine a plurality of target planes through the 3D volume to be extracted and recording said target planes in a target plane list;
perform a plane capturing operation comprising, for each received frame of the 3D ultrasound data:
extracting (22) a selected set of the determined plurality of target planes from the 3D ultrasound data;
controlling (28) a user interface to provide a real-time ultrasound image display output displaying a selected subset of the extracted set of planes of the current frame; and
storing the extracted planes in a plane dataset;
perform, in parallel with the plane capturing operation, a quality assessment operation comprising:
applying (24) a pre-determined quality assessment algorithm to planes in the plane dataset to determine, for each target plane in the target plane list, whether any individual extracted plane in the plane dataset corresponding to the target plane meets a pre-defined quality standard, or whether a time-series of extracted planes in the plane dataset corresponding to the same target plane meets a pre-defined quality standard, and
updating (26) the target plane list to remove from the list any of the target planes which has met the pre-determined quality standard; and
repeat the steps of the plane capturing operation for each next 3D frame of the series of frames until the target plane list is empty,
wherein, for each frame, the selected set of target planes to be extracted comprises at least each of the planes still included on the list;
wherein, for each frame, the selected subset of the extracted planes to be displayed on the user interface includes at least one of the planes still included on the list.

12. A system (30), comprising:
the processing device (32) of claim 11; and
an ultrasound acquisition system (54) comprising an ultrasound probe for manipulation by a user.

13. The system of claim 12, further comprising a user interface (52) comprising a display unit.
